(19) Europäisches Patentamt
European Patent Office
Office européen des brevets

(11) Publication number: **0 014 509**
A2

(12) # EUROPEAN PATENT APPLICATION

(21) Application number: 80200088.5

(22) Date of filing: 01.02.80

(51) Int. Cl.[3]: **A 61 K 7/48**

(30) Priority: 08.02.79 US 10334

(43) Date of publication of application: 20.08.80 Bulletin 80/17

(84) Designated Contracting States: AT BE CH DE FR GB IT LU NL SE

(71) Applicant: THE PROCTER & GAMBLE COMPANY
301 East Sixth Street
Cincinnati Ohio 45202(US)

(72) Inventor: Snyder, William Earl
6772 Menz Lane
Cincinnati, Ohio 45238(US)

(74) Representative: Gibson, Tony Nicholas et al,
Procter & Gamble (NTC) Limited Whitley Road
Longbenton Newcastle upon Tyne NE12 9TS(GB)

(54) **Skin conditioning compositions.**

(57) Skin conditioning compositions comprising a long chain alkyl ester of a fatty acid, an emollient material, an emulsifier and water. The long chain alkyl ester and the emollient combine to provide a very occlusive, cosmeticly acceptable, film on skin thereby reducing moisture loss therefrom.

EP 0 014 509 A2

Croydon Printing Company Ltd.

# SKIN CONDITIONING COMPOSITIONS

The present invention relates to novel skin conditioning compositions which form a very water occlusive film on skin thereby reducing moisture loss from the skin surface.

The treatment of human skin with various agents has been undertaken for many years with the goal being to keep the skin in a smooth and supple condition. Skin has the tendency to dry out when exposed to conditions of low humidity or to extended periods in a detergent solution. From a biochemical standpoint, dryness is a measure of the water content of the skin. Under normal conditions, the water content and vapor pressure of the epidermis are higher than those of the surrounding air with consequent evaporation of water from the skin surface. Skin becomes dry because of the excessive loss of water from the surface and the subsequent loss of water from the stratum corneum.

Continuous and prolonged immersion in soap or detergent solutions may contribute to dryness of the stratum corneum. The reason for this is that the surfactant medium promotes dissolution of the skin surface lipids, the horny layer lipids, and the dissolution of the hygroscopic water-soluble components in the corneum.

To alleviate the aforementioned conditions, emollient creams as described in Sagarin, Cosmetics Science and Technology, 2nd Edition, Vol. 1, Wiley Interscience (1972) have been recommended for application to the skin. The emollient materials probably increase the state of hydration of the corneous layer of the skin by altering the rate of diffusion of water from the lower epidermal and dermal layers, the rate of evaporation of water from the skin's surface, and the ability of the corneous layer to hold moisture.

Numerous other references have disclosed the use of a wide variety of materials for alleviating the problems of dry skin. Included among such references are those in which long chain esters of the type used in the compositions of the present invention have been disclosed. U.S. Patent 3,959,491, May 25, 1976 to Young et al discloses a milk based cosmetic which may contain esters such as dodecyl stearate and other components including fatty acids and emulsifiers. Technical Bulletins SG-0202 and SG-0211 distributed by Scher Chemicals, Inc. have also disclosed skin treatment compositions containing long chain esters, alcohols and surfactants. However, none of these references teach or suggest the invention as set forth herein.

## DISCLOSURE OF THE INVENTION

The present invention relates to superior skin conditioning compositions comprising from about 0.1% to about 16% of a long chain alkyl ester of a fatty acid, an emollient in an amount such that the amount of long chain alkyl ester and the emollient is from about 0.2%

to about 25%, from about 0.05% to about 8% of an emulsifier and the remainder water. The materials from which the emulsifier and the emollient may be selected are discussed below.

## DETAILED DESCRIPTION OF THE INVENTION

It has been discovered that compositions comprising the above generally described components possess superior occlusive properties. The compositions, in addition to their occlusiveness, possess good cosmetics. The individual components are described in detail below.

## LONG CHAIN ALKYL ESTER OF A FATTY ACID

The ester component of the compositions of the present invention has the structure

$$R_1 - \overset{O}{\overset{\|}{C}} - O - R_2$$

wherein $R_1$ and $R_2$ are both straight chain alkyls having from about 6 to about 22 carbon atoms and the total number of carbon atoms in the ester is at least 24. Examples of such esters include lauryl laurate, lauryl myristate, myristyl myristate, behenyl caprate, cetearyl palmitate, behenyl stearate. Esters having a total number of carbon atoms in excess of 28 are preferred. Cetearyl palmitate and cetyl stearate are most preferred esters. There are, of course, many other esters which meet the above described requirements and are suitable for use in the present compositions. Cetearyl palmitate is defined in the CTFA Cosmetic Ingredient Dictionary, page 55, 2nd edition, edited by N.F. Estrin.

Each chain of the ester, as indicated above, may contain from about 6 to about 22 carbon atoms while the total number must be at least 24. Thus esters having substantially unequal numbers of carbon atoms in the two chains are suitable for use in the present compositions. Chains of approximately equal length are, however, preferred.

The level of the above esters used in the present compositions is from about 0.1% to about 16% of the total composition, preferably from about 1% to about 8%.

The combination of the esters with the other oil phase components is important to the performance of the compositions of the present invention. Given below are preferred minimum and maximum levels for certain of the esters expressed as a percentage of the oil phase.

| CARBON CHAIN LENGTH | MINIMUM % | MAXIMUM % |
|---|---|---|
| $C_{24}$ | 64 | >64 |
| $C_{26}$ | 21 | >64 |
| $C_{28}$ | 9.9 | >64 |
| $C_{30}$ | 6.0 | >64 |
| $C_{32}$ | 4.2 | >64 |
| $C_{34}$ | 3.2 | 35 |
| $C_{36}$ | 2.6 | 23.9 |
| $C_{38}$ | 2.3 | 20.0 |
| $C_{40}$ | 2.0 | 18.2 |
| $C_{42}$ | 1.8 | 17.2 |
| $C_{44}$ | 1.7 | 16.6 |

Emollient Material

The present compositions, in addition to the above described esters, contain an emollient material in an amount such that the amount of ester plus emollient is from about 0.2% to about 25% of the total composition, preferably from about 4% to about 18%. One function of the emollient is to ensure that the ester is plasticized sufficiently to allow it to be in a film-like state on the skin. The emollient in the present compositions is selected from the group consisting of fatty alcohols, squalane, liquid or solid paraffins, mixtures of fatty acids and squalane, mixtures of fatty acids and liquid or solid paraffins and mixtures thereof.

The particular emollient selected depends in part on the particular ester selected since proper plasticization, as indicated above, is desired. The emollient for the esters having more than 28 carbon atoms is preferably selected from the group consisting of squalane, liquid or solid paraffins and mixtures of fatty alcohols with squalane or paraffins.

Typical fatty alcohols and fatty acids useful in the present compositions include those having from 12 - 22 carbon atoms such as cetyl alcohol, myristyl alcohol, stearyl alcohol, stearic acid and palmitic acid. Paraffins include, for example, mineral oil, petrolatum and paraffin wax.

Emulsifier

An emulsifier in an amount of from about 0.05% to about 8%, preferably from about 0.25% to about 5.0%, is included in the compositions of the present invention to emulsify the oil components. The emulsifier is selected from the group consisting of polyethoxylated fatty acids having less than about 30 moles of ethylene oxide per

mole of fatty acid, unethoxylated sugar esters, polyoxyethylene fatty ether phosphates, fatty acid amides, phospholipids, polypropoxylated fatty ethers, acyl lactylates and mixtures thereof.

Examples of such emulsifiers include polyoxyethylene (8) stearate, methyl glucoside sesquistearate, sucrose distearate, sucrose laurate, sorbitan monolaurate, polyoxyethylene (3) oleyl ether phosphate, polyoxyethylene (10) oleyl ether phosphate, lauric diethanolamide, stearic monoethanolamide, lecithin, lanolin alcohol propoxylates, sodium stearoyl-2-lactylate and calcium stearoyl-2-lactylate.

Preferred emulsifiers are the polyethoxylated fatty acids having less than about 30 moles of ethylene oxide per mole of fatty acid and the acyl lactylates.

Water

It is preferred that distilled water be used in the present compositions.

Optional Components

Oil Phase Components

In addition to the long chain esters, emollients and emulsifiers described previously, the present compositions may contain a variety of materials including:

a) Esters not meeting the requirements for the long chain ester such as oleyl oleate, isostearyl isostearate, isopropyl lanolate, isopropyl myristate, butyl stearate, myristyl lactate and 2-ethyl hexyl palmitate;

b) Oils such as castor oil, jojoba oil, cottonseed oil, peanut oil, sesame oil and silicone oils such as dimethyl polysiloxane;

c) Waxes such as ceresin wax, carnuba wax, beeswax and castor wax;

d) Lanolin, its derivatives and components such as acetylated lanolin, lanolin alcohols and lanolin fatty acids. Lanolin fatty acids are described in Re. U.S. Patent 29,814, October 24, 1978 to W. E. Snyder incorporated herein by reference.

e) Polyalkylenes such as hydrogenated polyisobutene and polyethylene; and

f) Sterols such as cholesterol and phytosterol.

These optional oil phase materials may comprise up to about 80% of the oil phase, preferably up to about 35%. When used at these levels, the optional components do not impair the occlusive nature of the compositions and add to the composition's total cosmetic performance.

Water Phase Components

The water phase of the compositions may contain many different materials including:

a) Humectants, such as sorbitol, glycerine, propylene glycol, alkoxylated glucose and hexanetriol at a level of from about 1% to about 20%;

b) Thickening agents such as carboxyvinyl polymers, ethyl cellulose, polyvinyl alcohol, carboxymethyl cellulose, vegetable gums and clays such as Veegum® (magnesium aluminum silicate, R. T. Vanderbilt, Inc.) at a level of from about 0.01% to about 6%;

c) Proteins and polypeptides at a level of from about 0.1% to about 3%;

d) Preservatives such as the methyl, ethyl, propyl and butyl esters of hydroxybenzoic acid (Parabens - Mallinckrodt Chemical Corporation) EDTA and imidazolidinyl ureas (Germall 115 - Sutton Laboratories) at a level of from about 0.2% to about 2.5%; and

e) An alkaline agent such as sodium hydroxide to neutralize, if desired, part of the fatty acids or thickener which may be present.

The present compositions may also contain agents suitable for aesthetic purposes such as perfumes and dyes. These agents may be used in amounts sufficient to achieve the desired fragrance and color (e.g. from about 0.05% to about 1%).

The compositions of the present invention are preferably substantially free of materials which adversely affect the esters' performance. Therefore, such things as polyethylene glycols and soaps are preferably present only at levels below about 1% of the total composition.

The pH of the present compositions is preferably in the range of about 4.5 - 9.0.

## Method of Manufacture

The compositions of the present invention generally have a lotion consistency and may be in the form of oil-in-water or water-in-oil emulsions with the former being preferred because of their more pleasing cosmetic properties.

The compositions of the present invention are preferably made by;

(A) preparing the oil phase;

(B) preparing the water phase; and

(C) adding the oil phase to the water phase.

Step (A) is carried out by heating the oil phase materials to a temperature of about 75°C. to about 100°C. Step (B) is carried out by heating the water phase materials to a temperature about the same as that of the oil phase. The emulsion is formed by slowly adding the oil phase prepared in step (A) to the water phase prepared in step (B) with stirring. Other ingredients may be added to the phase in which they are soluble prior to the mixing of the two phases or added directly to the mixed water and oil phases.

## Industrial Applicability

The compositions of the present invention are useful in the skin care field generally.

The following examples further describe and demonstrate the preferred embodiments within the scope of the present invention. Said examples are given solely for the purpose of illustration and are not to be construed as limitations of the present invention, as many variations thereof are possible without departing from the spirit and scope thereof. Unless otherwise indicated, all percentages herein are by weight.

All of the examples are shown as an oil phase and a water phase with the percentages being those of the total composition.

## EXAMPLE I

**Oil Phase**

| Materials | Wt.% |
|---|---|
| Amerlate WFA® (lanolin fatty acids)[1] | 0.50 |
| Stearic Acid | 0.75 |
| Crodamol CSP (cetearyl palmitate)[2] | 1.25 |
| Cetyl Alcohol | 2.50 |
| Robane® (Squalane)[3] | 1.25 |
| Petrolatum | 1.00 |
| Lecithin | 0.75 |
| Pationic SSL® (sodium stearoyl-2-lactylate)[4] | 1.25 |

**Water Phase**

| Materials | Wt.% |
|---|---|
| Distilled Water | 85.60 |
| Glycerin | 4.00 |
| Xanthan Gum | 0.30 |
| Methyl Paraben[5] | 0.25 |
| Propyl Paraben[5] | 0.10 |
| EDTA·4Na | 0.10 |
| $TiO_2$ | 0.20 |
| Perfume | 0.20 |

1. Supplied by Amerchol, a unit of CPC, International, Inc.
2. Supplied by Croda, Inc.
3. Supplied by Robeco Chemicals, Inc.
4. Supplied by Patco Cosmetic Products
5. Supplied by Mallinckrodt Chemical Corporation

EXAMPLE II

Oil Phase

| Materials | Wt.% |
|---|---|
| Amerlate LFA® (lanolin fatty acids) | 0.50 |
| Palmitic Acid | 0.75 |
| Cetyl Palmitate | 2.50 |
| Light Mineral Oil | 0.75 |
| Pationic SSL® (sodium stearoyl-2-lactylate) | 0.75 |
| Glycerol Monostearate | 0.50 |
| Patlac IL® (isostearyl lactate)[1] | 0.75 |
| Amerlate P® (isopropyl ester of lanolin fatty acids)[2] | 1.00 |
| Stearyl Alcohol | 2.50 |

Water Phase

| Materials | Wt.% |
|---|---|
| Distilled Water | 79.05 |
| Sorbo | 4.25 |
| 1.5% Carbopol 934® (aqueous solution)[3] | 5.25 |
| 1% NaOH | 0.75 |
| Methyl Paraben | 0.20 |
| Propyl Paraben | 0.10 |
| $TiO_2$ | 0.20 |
| Perfume | 0.20 |

1. Supplied byPatco Cosmetic Products
2. Supplied by Amerchol
3. Carboxyvinyl polymer supplied by B. F. Goodrich Chemical Co.

## EXAMPLE III

### Oil Phase

| Materials | Wt.% |
|---|---|
| Cetyl Alcohol | 2.25 |
| Lauric Acid | 1.00 |
| Stearyl Stearate | 1.75 |
| Polysynlane (hydrogenated polyisobutene)[1] | 1.50 |
| Petrolatum | 1.25 |
| Castor Oil | 0.50 |
| Schercemol MEM-3 (myristyl ethoxy (3) myristate)[2] | 0.75 |
| Schercemol DICA (diisocetyl adipate) | 1.25 |
| Myrj 45® (POE (8) stearate)[4] | 0.60 |
| Pationic SSL (sodium stearoyl-2-lactylate) | 0.90 |

### Water Phase

| Materials | Wt.% |
|---|---|
| Distilled Water | 78.45 |
| Propylene Glycol | 2.25 |
| Sorbo (sorbitol solution) | 1.75 |
| 1.5% Carbopol 934® (aqueous solution) | 3.75 |
| 1% NaOH Solution | 1.25 |
| Methyl Paraben | 0.20 |
| Propyl Paraben | 0.10 |
| Germall 115 (imidazolidinyl urea)[5] | 0.10 |
| $TiO_2$ | 0.20 |
| Perfume | 0.20 |

1. Supplied by Polyesther Corp.
2. Supplied by Scher Chemicals, Inc.
3. Supplied by Scher Chemicals, Inc.
4. Supplied by Atlas Chemicals, a division of ICI America, Inc.
5. Supplied by Sutton Laboratories, Inc.

EXAMPLE IV

Oil Phase

| Materials | Wt. % |
| --- | --- |
| Cetearyl Palmitate | 1.80 |
| Myristyl Myristate | 0.50 |
| Stearic Acid | 0.50 |
| Amerlate LFA® (lanolin fatty acids) | 0.50 |
| Oleyl Oleate | 1.00 |
| Cetyl Alcohol | 3.50 |
| Petrolatum | 0.80 |
| Patlac IL (isostearoyl lactate)[4] | 0.70 |
| Silicone 200 Fluid (polydimethylsiloxane - 350 centistokes)[1] | 0.50 |
| Pationic ISL (sodium isostearoyl lactylate)[4] | 0.90 |
| Crodafos N-3 (complex oleyl ether phosphate w/3 mole ethoxylation)[2] | 0.30 |

Water Phase

| Materials | Wt.% |
| --- | --- |
| Distilled Water | 73.30 |
| Glucam E-20® (alkoxylated glucose)[3] | 4.50 |
| Sorbo (70% sorbitol aqueous solution) | 3.00 |
| 1.5% Carbopol 934® (carboxyvinyl polymer) | 5.00 |
| 1% NaOH solution | 2.40 |
| Methyl Paraben | 0.30 |
| Propyl Paraben | 0.10 |
| $TiO_2$ | 0.20 |
| Perfume | 0.20 |

1. Supplied by Dow Corning Corporation
2. Supplied by Croda, Inc.
3. Supplied by Amerchol a unit of CPC, International, Inc.
4. Patco Cosmetic Products

EXAMPLE V

The four preceding compositions were prepared following the outline below.

1. Oil phase ingredients are placed in a suitable container and heated to 165°F. (73.9°C.).

2. When completely melted they are thorougly mixed.

3. The water phase ingredients, with the exception of perfume, neutralizing agents (NaOH, TEA, etc.), preservatives (Parabens, Germall, EDTA) and pigments ($TiO_2$) are also heated, in a separate container, to 165°F., with mechanical agitation.

4. When both phases are up to the proper temperature, the oil phase is added to the water phase with continued agitation.

5. If the formula contains a neutralizing agent, it is added at this time.

6. After the emulsion has been formed, the preservatives and pigment (if present in the formula) are added.

7. Mixing at moderate shear is continued for 20 minutes.

8. Temperature is lowered, with continued agitation, to 120°F., at which point the perfume is added.

9. After a 10 minute period of mixing, to incorporate the perfume, the mixture is cooled to 80°F. and packed into containers.

CLAIMS

1. A skin conditioning composition characterized by:-

(a) from 0.1% to 16% of an ester having the structure

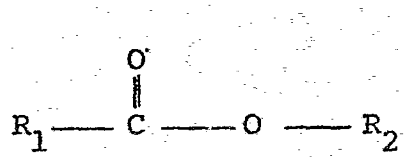

wherein $R_1$ and $R_2$ are both straight chain alkyls having from 6 to 22 carbon atoms and the total number of carbon atoms in the ester is at least 24;

(b) an emollient selected from the group consisting of fatty alcohols, squalane, liquid or solid paraffins, mixtures of fatty acids and squalane, mixtures of fatty acids and liquid or solid paraffins and mixtures thereof in an amount such that the amount of ester plus emollient is from 0.2% to 25%;

(c) from 0.05% to 8% of an emulsifier selected from the group consisting of polyethoxylated fatty acids having less than about 30 moles of ethylene oxide per mole of fatty acid, unethoxylated sugar esters, polyoxyethylene fatty ether phosphates, fatty acid amides, phospholipids, polypropoxylated fatty ethers, acyl lactylate salts and mixtures thereof; and

(d) water.

2. A skin conditioning composition according to Claim 1 characterized in that the ester is present at a level of from 1% to 8% and the total number of carbon atoms in the ester is in excess of 28.

3. A skin conditioning composition according to Claim 1 or 2 characterized in that the emollient is selected from the group consisting of squalane, liquid or solid paraffins, mixtures of fatty alcohols and squalane and mixtures of fatty alcohols and liquid or solid paraffins.

4. A skin conditioning composition according to any of Claims 1 to 3 characterized in that the emulsifier is selected from the group consisting of polyethoxylated fatty acids having less than 30 moles of ethylene oxide per mole of fatty acid and acyl lactylates.

5. A skin conditioning composition according to any of Claims 1 to 4 characterized in that the emulsifier is present at a level of 0.25% to 5.0%

6. A skin conditioning composition according to any of Claims 1 to 5 characterized in that in addition it contains from 1% to 20% of a humectant.

7. A skin conditioning composition according to any of Claims 1 to 6 characterized in that in addition it contains additional oil phase components at a level of up to 80% of the total level of oil phase components.

8. A skin conditioning composition according to any of Claims 1 to 7 characterized in the ester is selected from the group consisting of cetearyl palmitate and cetyl stearate.

9. A skin conditioning composition according to any of Claims 1 to 8 characterized in that in addition it contains a thickening agent.

10. A skin conditioning composition according to any of Claims 1 to 9 characterized in that it is in the form of an oil-in-water emulsion.